Europäisches Patentamt

European Patent Office

Office européen des brevets

(11) Veröffentlichungsnummer : **0 420 035 B1**

## (12) EUROPÄISCHE PATENTSCHRIFT

(45) Veröffentlichungstag der Patentschrift :
**04.08.93 Patentblatt 93/31**

(21) Anmeldenummer : **90118080.2**

(22) Anmeldetag : **20.09.90**

(51) Int. Cl.⁵ : **C07C 29/141,** C07C 29/145,
C07C 31/125, C07C 29/17

### (54) Verfahren zur Herstellung von Alkoholen (zweistufig).

(30) Priorität : **28.09.89 DE 3932331**

(43) Veröffentlichungstag der Anmeldung :
**03.04.91 Patentblatt 91/14**

(45) Bekanntmachung des Hinweises auf die
Patenterteilung :
**04.08.93 Patentblatt 93/31**

(84) Benannte Vertragsstaaten :
**AT BE CH DE ES FR GB IT LI NL SE**

(56) Entgegenhaltungen :
**DE-A- 3 530 839
DE-B- 1 276 618
SU-A- 338 045**

(73) Patentinhaber : **HOECHST
AKTIENGESELLSCHAFT
W-6230 Frankfurt am Main 80 (DE)**

(72) Erfinder : **Horn, Gerhardt, Dr. Dipl.-Chem.
Bunsenstrasse 19
W-4200 Oberhausen 11 (DE)**
Erfinder : **Frohning, Carl-Dieter, Dr.
Dipl.-Chem.
Regnitstrasse 50
W-4230 Wesel (DE)**
Erfinder : **Liebern, Hans
Hofacker 18
W-4330 Mülheim/Ruhr (DE)**
Erfinder : **Zgorzelski, Wolfgang, Dipl.-Ing.
Thüringer Strasse 20
W-4200 Oberhausen 11 (DE)**

## Beschreibung

Die vorliegende Erfindung betrifft ein zweistufiges Verfahren zur Herstellung von Alkoholen ausgehend von organischen Carbonylverbindungen. Es ist bekannt, organische Carbonylverbindungen bei erhöhter Temperatur und gegebenenfalls erhöhtem Druck mit Wasserstoff in Gegenwart eines Hydrierkatalysators in zwei oder mehr Stufen zu den entsprechenden Alkoholen umzusetzen. Die Umsetzung kann in jeder dieser Stufen sowohl diskontinuierlich als auch kontinuierlich in homogener oder heterogener Phase durchgeführt werden. Dementsprechend liegt der Hydrierkatalysator entweder in gelöster Form oder feinteilig als Suspension oder stückig als Festbettkontakt vor. Die zu hydrierenden Carbonylverbindungen können dem Katalysator in gasförmigem oder flüssigem Zustand zugeleitet werden. Erfindungsgemäß werden die Carbonylverbindungen in einer ersten Stufe in gasförmigem Zustand und in einer zweiten Stufe in flüssigem Zustand zur Reaktion gebracht.

Eine umfassende Darstellung bezüglich der Herstellung von Alkoholen durch katalytische Hydrierung von Carbonylverbindungen, insbesondere von Ketonen, Aldehyden und deren Derivaten findet sich in Houben-Weyl, Methoden der organischen Chemie, Georg Thieme Verlag Stuttgart-New York 1984, Band VI/1 b Seiten 9 bis 111.

Die DE-AS 12 27 882 beschreibt eine zwei- oder dreistufige Hydrierung ungesättigter Aldehyde in der Gasphase, wobei in der ersten Stufe ein Kupferkatalysator und in der zweiten Stufe ein Palladiumkatalysator oder in der zweiten Stufe ein Nickelträgerkatalysator und in der dritten Stufe ein Palladiumkatalysator verwendet wird.

Die DE-AS 12 76 618 betrifft eine zweistufige Hydrierung gesättigter und ungesättigter Aldehyde in der Gasphase, wobei die Reaktion zunächst an einem Kupfer-Nickel-Katalysator und nachfolgend an einem Nickel- und/oder Palladiumkatalysator abläuft.

Im Zusammenhang mit der Herstellung von 2-Ethylhexanol ausgehend von Propylen beschreibt die DE 35 30 839 Al eine zweistufige Hydrierung von 2-Ethylhexenal, wobei die Umsetzung zuerst in der Gasphase unter Verwendung eines Kupferkatalysators und anschließend in flüssiger Phase unter Einsatz eines nickelhaltigen Katalysators durchgeführt wird.

Die vorstehend beschriebenen Verfahren zur Herstellung von Alkoholen lassen hinsichtlich Umsatz und/oder Selektivität der katalytischen Hydrierungen Wünsche offen. Wegen der relativ langen Reaktionszeiten ergeben sich entsprechend niedrige Durchsätze bei hohen Reaktionstemperaturen, insbesondere in der zweiten Stufe. Aus dem erhaltenen Reaktionsprodukt läßt sich das Wertprodukt zumeist nur mit einem großen Aufwand destillativ in der gewünschten Reinheit gewinnen.

Daher besteht ein Bedarf nach einem Verfahren, das die vorstehend genannten Nachteile beseitigt. Diese Aufgabe wird gelöst durch ein Verfahren zur Herstellung von Alkoholen durch Umsetzung von organischen Carbonylverbindungen bei erhöhter Temperatur und gegebenenfalls erhöhtem Druck mit Wasserstoff in zwei Stufen, wobei die organischen Carbonylverbindungen in der ersten Stufe in gasförmigem Zustand einem kupferhaltigen Katalysator und in der zweiten Stufe in flüssigem Zustand einem nickelhaltigen Katalysator zugeführt werden.

Es ist dadurch gekennzeichnet, daß die organischen Carbonylverbindungen in der ersten Stufe zu 80 bis 99,5% der Theorie und in der zweiten Stufe mittels eines Nickel-Aluminiumoxid-Zirkondioxid enthaltenden Trägerkatalysators umgesetzt werden.

Das erfindungsgemäße Verfahren ermöglicht es, die Hydrierung in beiden Stufen mit gegenüber bekannten Verfahren erheblich gesteigertem Durchsatz zu betreiben. Dabei wird zugleich sichergestellt, daß die Gesamtreaktion mit sehr hohem Umsatz und sehr großer Selektivität abläuft. Ferner ist hervorzuheben, daß die Hydrierung in der zweiten Stufe trotz eines erhöhten Anteils von in der ersten Stufe nicht umgesetzten Carbonylverbindungen bei relativ niedrigen Temperaturen durchzuführen ist. Das gewonnene Reaktionsprodukt läßt sich mit vergleichsweise geringem destillativen Aufwand in ein Endprodukt hoher Reinheit überführen.

Das erfindungsgemäße Verfahren läßt sich auf die Umsetzung von Ketonen, Ketonderivaten, Aldehyden und deren Derivaten anwenden.

Als Ketone können Aceton, Methylethylketon, Diethylketon, Hexanone, beispielsweise Cyclohexanon, Heptanone, Octanone, aber auch höhere Ketone sowie aromatische Ketone, wie Acetophenon, Benzophenon, als Ketonderivate Acetol (Hydroxyaceton), Acetoin (Acetylmethylcarbinol), Dihydroxyaceton, Benzoin, Lactone sowie Ketosen, wie Fructose eingesetzt werden.

Mit Hilfe des erfindungsgemäßen Verfahrens lassen sich aromatische, araliphatische, cycloaliphatische und aliphatische Aldehyde und deren Derivate, insbesondere cycloaliphatische und aliphatische, bevorzugt aliphatische Aldehyde und deren Derivate umsetzen. Besondere Bedeutung kommt dem Gebrauch gesättigter und ungesättigter aliphatischer Aldehyde mit 2 bis 18 C-Atomen zu. Mit Ausnahme von Acetaldehyd lassen sich diese Aldehyde beispielsweise durch Hydroformylierung von Olefinen oder Aldolkondensation zweier glei-

cher oder verschiedener Aldehyde herstellen. Sie können in vorgereinigter Form, aber auch als rohes Reaktionsgemisch in das erfindungsgemäße Verfahren eingesetzt werden.

Geeignete gesättigte Aldehyde sind: Acetaldehyd, Propanal, n- und i-Butanal, n- und i-Pentanal, n- und i-Hexanal, n- und i-Heptanal, n- und i-Octanal, n- und i-Nonanal, n- und i-Decanal sowie n- und i-Alkanale mit 11 bis 18 Kohlenstoffatomen, insbesondere Acetaldehyd, Propanal, n- und i-Butanal, n- und i-Pentanal, n- und i-Octanal, n- und i-Nonanal sowie Alkanale mit 11 bis 18 Kohlenstoffatomen, bevorzugt Propanal, n- und i-Butanal sowie n- und i-Octanal.

Erfindungsgemäß lassen sich auch ungesättigte Aldehyde umsetzen. Hierzu zählen: Acrolein, Crotonaldehyd, Methacrolein, Ethylacrolein, Propylacrolein, Heptenal, Octenal, beispielsweise 2-Ethylhexenal, insbesondere Crotonaldehyd, 2-Ethylhexenal, bevorzugt 2-Ethylhexenal.

Die erfindungsgemäße Arbeitsweise eignet sich besonders als kontinuierliches Verfahren. Der die Carbonylverbindungen enthaltende Einsatzstoff wird in einem Verdampfer erhitzt und in gasförmigem Zustand zusammen mit Wasserstoff der ersten Stufe zugeleitet. Der kupferhaltige Katalysator wird in stückiger Form verwendet. Üblicherweise liegt er als Schicht angeordnet in einem senkrecht stehenden Rohrreaktor vor, der mit einer Kühl- und Heizvorrichtung ausgestattet ist. Der Einsatzstoff kann zusammen mit Wasserstoff entweder von unten nach oben oder vorzugsweise von oben nach unten über die Katalysatorschicht geführt werden.

Man arbeitet bei 100 bis 200, insbesondere 120 bis 180, bevorzugt 140 bis 160°C und bei 0,05 bis 2,0, insbesondere 0,1 bis 1,2, bevorzugt 0,15 bis 1,0 MPa. Temperatur und Druck richten sich auch nach der Art des Einsatzstoffes. Reaktionsfähige Carbonylverbindungen lassen sich bereits bei relativ niedrigen Temperaturen von 100 bis 130°C umsetzen, während reaktionsträge Carbonylverbindungen höhere Temperaturen, beispielsweise 150 bis 180°C erfordern. In den meisten Fällen hat sich ein Temperaturbereich von 140 bis 160°C als geeignet erwiesen. Die Reaktionstemperatur wird auch von der Nutzungsdauer des Katalysators beeinflußt. So ermöglicht ein frischer, ungebrauchter Katalysator eine Umsetzung der Carbonylverbindungen bei niedrigen Temperaturen, beispielsweise bei 130 bis 145°C, während ein gebrauchter Katalysator in Abhängigkeit von der Nutzungsdauer allgemein höhere Temperaturen, beispielsweise oberhalb 150, insbesondere oberhalb 155°C, zur Durchführung der Hydrierung benötigt.

Der in der ersten Stufe verwendete kupferhaltige Katalysator enthält 15 bis 85, insbesondere 30 bis 80, bevorzugt 50 bis 70 Gew.-% Kupfer, 0,2 bis 20, insbesondere 1,0 bis 10, bevorzugt 2 bis 8 Gew.-% MgO, 0,03 bis 12, insbesondere 0,5 bis 8, bevorzugt 1 bis 5 Gew.-% $Cr_2O_3$ und 5 bis 80, insbesondere 7 bis 60, bevorzugt 9 bis 50 Gew.-% $SiO_2$ als Träger jeweils bezogen auf die Katalysatorzusammensetzung.

Es stellt ein wesentliches Merkmal des erfindungsgemäßen Verfahrens dar, in der ersten Stufe die Carbonylverbindungen nicht soweit als möglich, sondern lediglich zum größten Teil und in der zweiten Stufe die noch vorhandenen Anteile der Carbonylverbindungen möglichst quantitativ zu den entsprechenden Alkoholen umzusetzen.

Man setzt die Carbonylverbindungen in der ersten Stufe zu 80 bis 99,5, insbesondere 90 bis 99, bevorzugt 93 bis 98% der Theorie um. Der Grad der Umsetzung ist von der Art des Einsatzstoffes, der jeweiligen Aktivität des Katalysators, der Temperatur und dem gewünschten Durchsatz abhängig.

Wasserstoff muß mindestens entsprechend der Stöchiometrie der Umsetzung eingesetzt werden. Üblicherweise verwendet man jedoch einen stöchiometrischen Überschuß an Wasserstoff, um die Reaktion in die gewünschte Richtung zu lenken. Ein Wasserstoffüberschuß von 0,5 bis 50, insbesondere 1 bis 20, bevorzugt 2 bis 10 Mol je Äquivalent Carbonylverbindung erweist sich für die Gasphasehydrierung in der ersten Stufe als ausreichend. Nichtumgesetzter Wasserstoff kann der Reaktion wieder zugeführt werden.

Das die erste Stufe verlassende Reaktionsgemisch wird kondensiert und der Wasserstoff gegebenenfalls abgetrennt. Es kann der zweiten Stufe des erfindungsgemäßen Prozesses entweder nach Zwischenlagerung oder unmittelbar zugeleitet werden.

In der zweiten Stufe erfolgt die Hydrierung in flüssiger Phase, wobei der Nickel, Aluminiumoxid und Zirkondioxid als Copräzipitat enthaltende Trägerkatalysator entweder als feinteilige Suspension oder als stückiger Festbettkontakt eingesetzt werden kann. Die Reaktionstemperatur beträgt 60 bis 150, insbesondere 80 bis 140, bevorzugt 90 bis 130, besonders bevorzugt 100 bis 125°C und der Druck 0,1 bis 25, insbesondere 1,0 bis 15, bevorzugt 2,0 bis 10 MPa.

Die Reaktionsbedingungen, nämlich die Temperatur und der Druck, hängen - wie bereits im Zusammenhang mit dem in der ersten Stufe verwendeten Katalysator beschrieben von der Art des Einsatzstoffes, dem Restgehalt an Carbonylverbindung, der Aktivität des Katalysators und dem gewünschten Durchsatz ab.

Reaktive Carbonylverbindungen lassen sich bereits bei vergleichsweise niedrigen Reaktionstemperaturen von 60 bis 120°C hydrieren, hingegen erfordern reaktionsträge Carbonylverbindungen höhere Temperaturen, beispielsweise 100 bis 150°C. In den meisten Fällen hat sich ein Temperaturbereich von 100 bis 125°C als geeignet erwiesen. Die Reaktionstemperatur wird auch von der Nutzungsdauer des Katalysators beeinflußt.

So ermöglicht ein frischer, ungebrauchter Katalysator eine Umsetzung der Carbonylverbindungen bei niedrigen Temperaturen, beispielsweise 80 bis 125°C, während ein gebrauchter Katalysator in Abhängigkeit von der Nutzungsdauer im allgemeinen höhere Temperaturen, beispielsweise oberhalb 110 insbesondere oberhalb 115, bevorzugt oberhalb 125°C zur Durchführung der Hydrierung benötigt.

Man leitet die umzusetzende flüssige Carbonylverbindung und Wasserstoff entweder dem in feinteiliger Form aufgeschlämmten Katalysator absatzweise oder kontinuierlich zu (Suspensionsfahrweise) oder führt den die Carbonylverbindung aufweisenden Einsatzstoff im Gleich- oder Gegenstrom mit Wasserstoff über den in stückiger Form vorliegenden, als Festbett angeordneten, Nickel, Aluminiumoxid und Zirkondioxid als Copräzipitat enthaltenden Trägerkatalysator. Bei technischer Realisierung des erfindungsgemäßen Verfahrens ist die Festbettfahrweise häufig zu bevorzugen, wobei das Einsatzgemisch entweder von oben nach unten (Rieselfahrweise) oder von unten nach oben (Sumpffahrweise) über den festangeordneten Katalysator geleitet wird. Wendet man die Rieselfahrweise an, so leitet man den Wasserstoff im Gleich- oder Gegenstrom, bevorzugt im Gleichstrom zum Einsatzmaterial, will man hingegen die Sumpffahrweise praktizieren, so führt man vorteilhaft den Wasserstoff im Gleichstrom zum Einsatzgemisch über den stückigen, festangeordneten Trägerkatalysator.

Wasserstoff muß mindestens entsprechend der Stöchiometrie der Umsetzung eingesetzt werden. In der Regel wird man jedoch einen stöchiometrischen Überschuß an Wasserstoff verwenden, um die Reaktion günstig zu beeinflussen. Ein Wasserstoffüberschuß von 1 bis 100, insbesondere 2 bis 50, bevorzugt 5 bis 10 Mol je Äquivalent Carbonylverbindung genügt zur Durchführung der Hydrierung in flüssiger Phase. Nichtumgesetzter Wasserstoff kann in die Reaktion zurückgeführt werden.

Der in der zweiten Stufe verwendete Hydrierkatalysator enthält 20 bis 90 Gew.-% Nickel bezogen auf Katalysatorzusammensetzung, sowie 1 bis 30, insbesondere 3 bis 15, bevorzugt 4 bis 10 Gew.-Teile Aluminiumoxid und 0,5 bis 20, insbesondere 1 bis 10, bevorzugt 1,5 bis 5 Gew.-Teile Zirkondioxid jeweils bezogen auf 100 Gew.-Teile Nickel als Copräzipitat auf einem Trägermaterial.

Da die Ausübung des erfindungsgemäßen Verfahrens in der zweiten Stufe an diesen besonderen Katalysator gebunden ist, soll im folgenden dessen Herstellung näher erläutert werden.

Man vermischt eine wäßrige Ni-Al-Zr-Mischsalzlösung mit einer wäßrigen Lösung einer basischen Verbindung als Fällungsmittel, setzt die basische Verbindung in einem stöchiometrischen Überschuß von 5 bis 100 %, bezogen auf die zur quantitativen Fällung von Ni, Al und Zr erforderliche Menge, ein, fällt bei 60 bis 120°C und pH 7 bis 10 gleichzeitig Ni, Al und Zr und scheidet sie als Copräzipitat auf einem Trägermaterial ab.

Um einer unerwünschten Hydrolyse vorzubeugen und die Fällung günstig zu beeinflussen, empfiehlt es sich, der Mischsalzlösung freie Säure im Überschuß entsprechend einem Verhältnis $H^+ : Zr^{4+} = (2 \text{ bis } 40) : 1$, insbesondere $(3 \text{ bis } 30) : 1$, bevorzugt $(4 \text{ bis } 20) : 1$, zuzufügen. Die freie Säure wird durch Titration mit NaOH (Endpunkt pH = 0,8) bestimmt.

Als freie Säure können Salzsäure, Schwefelsäure und bevorzugt Salpetersäure eingesetzt werden.

Die Mischsalzlösung besteht aus 10 bis 100, insbesondere 20 bis 80, bevorzugt 30 bis 50 g Ni/l. Sie weist Aluminium entsprechend 1 bis 30, insbesondere 3 bis 15, bevorzugt 4 bis 10 Gew.-Teilen $Al_2O_3$ je 100 Gew.-Teile Ni auf. Ferner enthält sie Zirkon entsprechend 0,5 bis 20, insbesondere 1 bis 10, bevorzugt 1,5 bis 5 Gew.-Teilen $ZrO_2$ je 100 Gew.-Teile Ni.

Die Mischsalzlösung stellt man her, indem man wasserlösliche anorganische, organische oder komplexe Salze von Nickel, Zirkon und Aluminium, insbesondere deren Sulfate, Chloride, Acetate und Nitrate, bevorzugt deren Nitrate in Wasser löst.

Als Fällungsmittel dient eine wäßrige Lösung einer basischen Verbindung, insbesondere eine wäßrige Alkalicarbonat-, Alkalihydrogencarbonat-, Alkalihydroxid-, Ammoniumhydroxid- oder Ammoniumcarbonatlösung, die einen pH-Wert von 7,5 bis 13, insbesondere 8 bis 12, bevorzugt 9 bis 11, aufweist.

Recht gute Ergebnisse liefern wäßrige Lösungen mit 0,3 bis 1,5, insbesondere 0,8 bis 1,2 Mol Alkalicarbonat/l Lösung.

Um eine möglichst vollständige Fällung eines besonders homogenen Copräzipitats sicherzustellen, setzt man die basische Verbindung im stöchiometrischen Überschuß von 5 bis 100, insbesondere 10 bis 70, bevorzugt 20 bis 40%, jeweils bezogen auf die zur vollständigen Fällung von Ni, Al und Zr erforderliche Menge basische Verbindung, ein.

Die Fällung wird herbeigeführt, indem man entweder die Mischsalzlösung und das Fällungsmittel kontinuierlich zusammenführt und vermischt oder, gemäß einer bevorzugten Variante, das Fällungsmittel vorlegt und die Mischsalzlösung in das Fällungsmittel einleitet.

Das Trägermaterial kann mit der Mischsalzlösung und/oder mit dem Fällungsmittel in die Reaktion eingesetzt werden.

Es hat sich als besonders vorteilhaft erwiesen, zunächst die Mischsalzlösung und das Fällungsmittel miteinander zu vermischen und anschließend das Trägermaterial zuzusetzen.

Als Trägermaterial eignen sich Aktivkohle, Tonerde, Bimsstein, $\gamma$-$Al_2O_3$, $SiO_2$, Kieselgel, Kieselgur und Kieselerde. Besonders haben sich $SiO_2$, Kieselgel, Kieselgur und Kieselerde bewährt. Bevorzugt werden Kieselgur und $SiO_2$ in Form gefällter Kieselsäure eingesetzt.

Üblicherweise setzt man 6 bis 80, insbesondere 15 bis 65, bevorzugt 35 bis 50 Gew.-Teile Trägermaterial je 100 Gew.-Teile Ni ein.

Zur Herstellung homogener Copräzipitate hält man während der Fällung einen pH-Bereich von 7 bis 10, insbesondere 7,3 bis 9, bevorzugt 7,5 bis 8,5 und eine Temperatur von 60 bis 120, insbesondere 70 bis 110, bevorzugt 95 bis 105°C ein.

Nach Beendigung der Fällung wird gegebenenfalls nach Abkühlung filtriert, gewaschen, erforderlichenfalls geformt, anschließend getrocknet und reduziert.

Die Trocknung wird in einem Temperaturbereich zwischen 40 bis 120, insbesondere 50 bis 100 °C durchgeführt.

Die Reduktion mittels Wasserstoff erfolgt bei 300 bis 550 °C, wobei ein Reduktionsgrad von wenigstens 80 %, insbesondere wenigstens 90 %, bevorzugt 95 % und darüber anzustreben ist. Unter Reduktionsgrad versteht man Anteil Nickelmetall: Gesamtgehalt Nickel x 100 %.

In der zweiten Stufe werden - wie bereits erwähnt - mit Hilfe des zuvor beschriebenen Nickel, Aluminiumoxid und Zirkondioxid als Copräzipitat enthaltenden Trägerkatalysators die in der ersten Stufe nicht umgesetzten Anteile der Carbonylverbindungen mit hohem Durchsatz möglichst vollständig zu den entsprechenden Alkoholen umgesetzt.

Das erfindungsgemäße Verfahren läßt sich generell mit gesteigertem Durchsatz betreiben. Man führt das die Carbonylverbindungen aufweisende Einsatzmaterial der ersten Stufe mit einer Raumgeschwindigkeit (Volumen flüssiges Einsatzmaterial/Volumen Katalysator x Stunde = V/Vh) von 0,3 bis 2,0, insbesondere 0,5 bis 1,8, bevorzugt 0,6 bis 1,5 und der zweiten Stufe mit einer Raumgeschwindigkeit von 0,5 bis 2,5, insbesondere 0,7 bis 1,8, bevorzugt 0,8 bis 1,5 zu.

Die in der ersten Stufe gewählte Raumgeschwindigkeit beeinflußt auch die in der zweiten Stufe anzustrebende Raumgeschwindigkeit und umgekehrt. Wählt man in der ersten Stufe eine hohe Raumgeschwindigkeit, beispielsweise 1,5 bis 2,0, oder 1,0 bis 1,5, oder 0,6 bis 1,0, so wird man die zweite Stufe mit einer entsprechend angepaßten Raumgeschwindigkeit, beispielsweise 0,5 bis 1,0, oder 1,0 bis 1,6, oder 1,6 bis 2,4 betreiben, um eine möglichst hohe Ausbeute an Wertprodukt sicherzustellen. Je höher die Raumgeschwindigkeit in der ersten Stufe ist, desto tiefer ist sie in der zweiten Stufe anzusetzen. Umgekehrt zieht eine steigende Raumgeschwindigkeit in der zweiten Stufe eine entsprechende herabgesetzte Raumgeschwindigkeit in der ersten Stufe nach sich.

Die Raumgeschwindigkeit richtet sich auch nach der Art des Einsatzstoffes. Im allgemeinen lassen sich gesättigte Carbonylverbindungen, insbesondere Aldehyde mit höheren Raumgeschwindigkeiten umsetzen, während ungesättigte Carbonylverbindungen, insbesondere Aldehyde eine etwas niedrigere Raumgeschwindigkeit erfordern.

So sollte bei Verwendung von n-Butanal die Raumgeschwindigkeit in der ersten Stufe 0,6 bis 2,0, insbesondere 0,8 bis 1,8 und in der zweiten Stufe 0,7 bis 2,0, insbesondere 0,9 bis 1,7, bei Einsatz von 2-Ethylhexenal in der ersten Stufe 0,4 bis 2,0, insbesondere 0,5 bis 1,8 und in der zweiten Stufe 0,6 bis 1,8, insbesondere 0,8 bis 1,6 betragen.

Die nachfolgend aufgeführten Beispiele belegen die Erfindung, ohne sie zu begrenzen.

**Experimenteller Teil**

Beispiel 1

1. Stufe

In einem senkrecht stehenden Rohr befindet sich eine Schüttung von 1000 ml eines tablettierten Katalysators, der in aktiviertem Zustand 80 Gew.-% Cu, 4,0 Gew.-% MgO, 2,0 Gew.-% $Cr_2O_3$ und 12 Gew.-% $SiO_2$ als Träger enthält. Der umzusetzende Einsatzstoff wird in flüssigem Zustand einem Verdampfer zugeführt und anschließend in gasförmigem Zustand zusammen mit Wasserstoff über die Schüttung des obengenannten Katalysators geleitet.

```
Reaktionsbedingungen
     Einsatzstoff: 1600 ml 2-Ethylhexenal je Stunde
     Zusammensetzung:   Vorlauf              0,6 Gew.-%
                        2-Ethylhexenal      98,9 Gew.-%
                        2-Ethylhexanal        -  Gew.-%
                        2-Ethylhexanol        -  Gew.-%
                        Höhersieder          0,5
                        CO-Zahl              434  (mg KOH/g)
                        Iodzahl              226  (g J₂/100g)
     Wasserstoff:       2400 Nl H₂ je Stunde
     V/Vh*:             1,6
     Druck:             0,2 MPa
     Temperatur:        145°C
     Umsatz               92 % bezogen auf Aldehyd
                          91 % bezogen auf C-C-Doppelbindung


     *   Raumgeschwindigkeit (Volumen flüssiger
         Einsatzstoff/Volumen Katalysator   x   Stunde)
```

Iodzahl 226 (g $J_2$/100g)

Wasserstoff: 2400 Nl $H_2$ je Stunde

### 2. Stufe

Das nach Verlassen der ersten Stufe anfallende gasförmige Reaktionsgemisch wird kondensiert und in flüssigem Zustand weiterverarbeitet. In einem senkrecht stehenden Rohr befindet sich eine Schüttung von 1000 ml eines tablettierten Katalysators, der 100 Gew.-Teile Ni, 5 Gew.-Teile Aluminiumoxid und 3 Gew.-Teile Zirkondioxid als Copräzipitat und 40 Gew.-Teile $SiO_2$ als Träger enthält. Das aus der ersten Stufe stammende Reaktionsgemisch wird in flüssigem Zustand über die Schüttung des obengenannten Katalysators von oben nach unten geleitet (Rieselfahrweise).

Reaktionsbedingungen

Einsatzstoff:  900 ml Reaktionsgemisch der ersten
              Stufe je Stunde

Zusammensetzung:  Vorlauf              0,4 Gew.-%

                  2-Ethylhexenal       8,0 Gew.-%

                  2-Ethylhexanal       0,6 Gew.-%

                  2-Ethylhexanol       90,9 Gew.%

                  Höhersieder          0,2 Gew.-%

                  CO-Zahl              35,4 (mgKOH/g)

                  Iodzahl              18,3 (g $J_2$/100g)

Wasserstoff:      120 Nl $H_2$ je Stunde

V/Vh*:            0,9

Druck:            2,5 MPa

Temperatur:       125°C

Umsatz:           bezogen auf Aldehyd >99,9%

                  bezogen auf C-C-Doppelbindung >99,9%

                  CO-Zahl: <0,05 (mg KOH/g)

                  Iodzahl: <0,03 (g $J_2$/100g)

*   Raumgeschwindigkeit (Volumen flüssiger
    Einsatzstoff/Volumen Katalysator  x  Stunde)

## Beispiel 2

### 1. Stufe

Es wird wie in der 1. Stufe von Beispiel 1 gearbeitet, jedoch mit geänderter Menge Einsatzstoff

Reaktionsbedingungen

Einsatzstoff:  900 ml 2-Ethylhexenal je Stunde

Zusammensetzung:   siehe Beispiel 1

Wasserstoff:       2400 Nl $H_2$ je Stunde

V/Vh:              0,9

Druck:             0,2 MPa

Temperatur:        145°C

Umsatz:            bezogen auf Aldehyd 99%

                   bezogen auf C-C-Doppelbindung 98%

### 2. Stufe

Es wird wie in der 2. Stufe von Beispiel 1 gearbeitet, jedoch mit geänderter Menge Einsatzstoff.

7

Reaktionsbedingungen:

Einsatzstoff:  1600  ml Reaktionsgemisch der ersten
Stufe je Stunde

Zusammensetzung:  Vorlauf                 0,1 Gew.-%

2-Ethylhexanol         96,6 Gew.-%

2-Ethylhexanal          1,0 Gew.-%

2-Ethylhexenal          2,0 Gew.-%

Höhersieder             0,3

CO-Zahl                 4,4  (mgKOH/g)

Iodzahl                 4,5  (g $J_2$/100g)

Wasserstoff:   120 Nl $H_2$ je Stunde

V/Vh:          1,6

Druck:         2,5 MPa

Temperatur:    125°C

Umsatz:        bezogen auf Aldehyd >99,9%

bezogen auf C-C-Doppelbindung >99,9%

CO-Zahl: 0,04 (mg KOH/g)

Iodzahl: <0,03 (g $J_2$/100g)

## Beispiel 3

### 1. Stufe

Es wird wie in der 1. Stufe von Beispiel 1 gearbeitet, jedoch mit n-Butanal als Einsatzstoff

Reaktionsbedingungen

Einsatzstoff:          1500 ml n-Butanal je Stunde

Vorlauf          0,3 Gew.-%

n-Butanal        99,2 Gew.-%

n-Butanol         -  Gew.-%

Höhersieder      0,5 Gew.-%

CO-Zahl          776 (mg KOH/g)

Wasserstoff:   2400 Nl $H_2$ je Stunde

V/Vh:          1,5

Druck:         0,2 MPa

Temperatur:    145°C

Umsatz:        bezogen auf Aldehyd 94%

CO-Zahl 46,6 (mg KOH/g)

### 2. Stufe

Es wird wie in der 2. Stufe von Beispiel 1 gearbeitet, jedoch mit dem bei der Umsetzung von n-Butanal in der ersten Stufe anfallenden Reaktionsgemisch als Einsatzstoff.

Reaktionsbedingungen

```
Einsatzstoff:    1000 ml Reaktionsgemisch der ersten
                 Stufe je Stunde
Zusammensetzung:  Vorlauf        0,5 Gew.-%
                  n-Butanal      5,9 Gew.-%
                  n-Butanol     93,2 Gew.-%
                  Höhersieder    0,1 Gew.-%
                  CO-Zahl       46,6 (mg KOH/g)
Wasserstoff:      100 Nl H₂ je Stunde
V/Vh:             1,0
Druck:            8,0 MPa
Temperatur:       115°C
Umsatz:           bezogen auf Aldehyd >99,9%
                  CO-Zahl ≤ 0,7 (mg KOH/g)
```

**Beispiel 4**

1. Stufe

Es wird wie in der 1. Stufe von Beispiel 3 gearbeitet, jedoch mit geänderter Menge Einsatzstoff

```
Reaktionsbedingungen
    Einsatzstoff:     900 ml n-Butanal je Stunde
    Zusammensetzung:  wie in Beispiel 3
    Wasserstoff:      2400 Nl H₂ je Stunde
    V/Vh:             0,9
    Druck:            0,2 MPa
    Temperatur:       145°C
    Umsatz:           bezogen auf Aldehyd 98,4%
                      CO-Zahl 12,4 (mg KOH/g)
```

2. Stufe

Es wird wie in der 2. Stufe von Beispiel 3 gearbeitet jedoch mit dem bei der Umsetzung von n-Butanal in der ersten Stufe anfallenden Reaktionsgemisch als Einsatzstoff.

9

Reaktionsbedingungen

| Einsatzstoff: | 1700 ml Reaktionsgemisch der ersten Stufe je Stunde |
|---|---|
| Zusammensetzung: | Vorlauf 0,2 Gew.-% |
| | n-Butanal 1,6 Gew.-% |
| | n-Butanol 98,0 Gew.-% |
| | Höhersieder 0,1 Gew.-% |
| | CO-Zahl 12,4 (mg KOH/g) |

| Wasserstoff: | 120 Nl $H_2$ je Stunde |
|---|---|
| V/Vh: | 1,7 |
| Druck: | 2,5 MPa |
| Temperatur: | 125°C |
| Umsatz: | bezogen auf Aldehyd $\geq$99,9% |
| | CO-Zahl $\leq$0,7 (mg KOH/g) |

Vergleichsbeispiel 1

1. Stufe

Es wird wie in der 1. Stufe von Beispiel 2 gearbeitet.

Reaktionsbedingungen

| Einsatzstoff: | 900 ml 2-Ethylhexenal je Stunde |
|---|---|
| Zusammensetzung: | siehe 1. Stufe von Beispiel 1 |
| Wasserstoff: | 2400 Nl $H_2$ je Stunde |
| V/Vh: | 0,9 |
| Druck: | 0,2 MPa |
| Temperatur: | 145°C |
| Umsatz: | bezogen auf Aldehyd: 99% |
| | bezogen auf C-C-Doppelbindung 98% |
| | CO-Zahl: 4,4 (mg KOH/g) |
| | Iodzahl: 4,5 (g $J_2$/100g) |

2. Stufe

Es wird wie in der 2. Stufe von Beispiel 1 gearbeitet, jedoch ein Nickelkatalysator mit etwa 55 Gew.-% Ni und etwa 30 bis 35 Gew.-% $SiO_2$, der allerdings weder Aluminiumoxid noch Zirkondioxid enthält, verwendet.

Reaktionsbedingungen

```
Einsatzstoff          900 ml Reaktionsgemisch der
                      1. Stufe je Stunde
Zusammensetzung:      Vorlauf              0,1  Gew.-%
                      2-Ethylhexanal       1,0  Gew.-%
                      2-Ethylhexenal       2,0  Gew.-%
                      2-Ethylhexanol      96,6  Gew.-%
                      Höhersieder          0,3
                      CO-Zahl              4,4  (mg KOH/g)
                      Iodzahl              4,5  (g J₂/100g)
```

$$Iodzahl \quad 4,5 \quad (g\ J_2/100g)$$

```
Wasserstoff:          120 Nl H₂ je Stunde
V/Vh:                 0,9
Druck:                2,5 MPa
Temperatur:           125°C
Umsatz:               bezogen auf Aldehyd 99,4%
                      bezogen auf C-C-Doppelbindung 99,3%
                      CO-Zahl 0,03
                      Iodzahl 0,03
```

Trotz eines gegenüber der 2. Stufe von Beispiel 1 bzw. gegenüber der 1. Stufe von Beispiel 2 reduzierten Durchsatzes (V/Vh) wird ein Endprodukt erhalten, das nicht die Qualität des in den Beispielen 1 und 2 erfindungsgemäß erhaltenen Endproduktes erreicht. Während das Reaktionsprodukt der Beispiele 1 und 2 sich ohne großen Aufwand zu einem 2-Ethylhexanol, das zur Herstellung von Weichmachern geeignet ist, verarbeiten läßt, gelingt dies mit dem Reaktionsprodukt des Vergleichsbeispiels 1 nicht.

Vergleichsbeispiel 2

1. Stufe

Es wird wie in der 1. Stufe von Beispiel 4 gearbeitet.

Reaktionsbedingungen

```
Einsatzstoff:         900 ml n-Butanal je Stunde
Zusammensetzung:      wie in der 1. Stufe von Beispiel 3
Wasserstoff:          2400 Nl H₂ je Stunde
V/Vh:                 0,9
Druck:                0,2 MPa
Temperatur:           145°C
Umsatz:               bezogen auf Aldehyd 98,4%
                      CO-Zahl 12,4
```

EP 0 420 035 B1

2. Stufe

Es wird wie in der 2. Stufe von Beispiel 3 gearbeitet, jedoch ein Nickelkatalysator mit etwa 55 Gew.-% Ni und etwa 30 bis 35 Gew.-% $SiO_2$, der allerdings weder Aluminiumoxid noch Zirkondioxid enthält, verwendet.

```
Reaktionsbedingungen
    Einsatzstoff:        1000 ml Reaktionsgemisch der 1.
                         Stufe von Vergleichsbeispiel 2 je
                         Stunde
    Wasserstoff:         100 Nl H₂ je Stunde
    V/Vh:                1,0
    Druck:               8,0 MPa
    Temperatur:          115°C
    Umsatz:              bezogen auf Aldehyd 99,4%
```

Im Vergleich hierzu belegt die erfindungsgemäße Arbeitsweise von Beispiel 4 nicht nur einen erheblich höheren Durchsatz in der 2. Stufe, sondern zugleich auch einen besseren Umsatz zum gewünschten Wertprodukt.

**Patentansprüche**

1. Verfahren zur Herstellung von Alkoholen durch Umsetzung von organischen Carbonylverbindungen bei erhöhter Temperatur und gegebenenfalls erhöhtem Druck mit Wasserstoff in zwei Stufen, wobei die organischen Carbonylverbindungen in der ersten Stufe in gasförmigem Zustand einem kupferhaltigen Katalysator und in der zweiten Stufe in flüssigem Zustand einem nickelhaltigen Katalysator zugeführt werden, dadurch gekennzeichnet, daß die organischen Carbonylverbindungen in der ersten Stufe zu 80 bis 99,5% der Theorie und in der zweiten Stufe mittels eines Nickel-Aluminiumoxid-Zirkondioxid enthaltenden Trägerkatalysators umgesetzt werden.

2. Verfahren nach Anspruch 1, dadurch gekennzeichnet, daß als Carbonylverbindungen Ketone, Ketonderivate, Aldehyde und Aldehydderivate eingesetzt werden.

3. Verfahren nach einem oder mehreren der Ansprüche 1 bis 2, dadurch gekennzeichnet, daß als Carbonylverbindungen Aldehyde und Aldehydderivate, insbesondere gesättigte und ungesättigte aliphatische Aldehyde eingesetzt werden.

4. Verfahren nach einem oder mehreren der Ansprüche 1 bis 3, dadurch gekennzeichnet, daß als Carbonylverbindungen n-Butanal und 2-Ethylhexenal eingesetzt werden.

5. Verfahren nach einem oder mehreren der Ansprüche 1 bis 4, dadurch gekennzeichnet, daß der kupferhaltige Katalysator 15 bis 85 Gew.-% Kupfer, 0,2 bis 20 Gew.-% MgO, 0,03 bis 12 Gew.-% $Cr_2O_3$ und 5 bis 80 Gew.-% $SiO_2$ als Träger jeweils bezogen auf die Katalysatorzusammensetzung enthält.

6. Verfahren nach einem oder mehreren der Ansprüche 1 bis 5, dadurch gekennzeichnet, daß in der ersten Stufe die Temperatur 100 bis 200°C und der Druck 0,05 bis 2,0 MPa und in der zweiten Stufe die Temperatur 60 bis 150°C und der Druck 0,1 bis 25 MPa beträgt.

7. Verfahren nach einem oder mehreren der Ansprüche 1 bis 6, dadurch gekennzeichnet, daß die Carbonylverbindungen in der ersten Stufe zu 90 bis 99,0, insbesondere 93 bis 98 % der Theorie umgesetzt werden.

8. Verfahren nach einem oder mehreren der Ansprüche 1 bis 7, dadurch gekennzeichnet, daß der Hydrierkatalysator 20 bis 90 Gew.-% Nickel bezogen auf Katalysatorzusammensetzung, sowie 1 bis 30 Gew.-Teile Aluminiumoxid und 0,5 bis 20 Gew.-Teile Zirkondioxid jeweils bezogen auf 100 Gew.-Teile Ni als Copräzipitat auf einem Trägermaterial enthält.

9. Verfahren nach einem oder mehreren der Ansprüche 1 bis 8, dadurch gekennzeichnet, daß der Nickel-Aluminiumoxid-Zirkondioxid enthaltende Trägerkatalysator als Trägermaterial Aktivkohle, Tonerde, Bimsstein, $\gamma$-Al$_2$O$_3$, SiO$_2$, Kieselgel, Kieselgur und/oder Kieselerde, insbesondere SiO$_2$, Kieselgel, Kieselgur und/oder Kieselerde, bevorzugt Kieselgur und/oder SiO$_2$ in Form gefällter Kieselsäure enthält.

10. Verfahren nach einem oder mehreren der Ansprüche 1 bis 9, dadurch gekennzeichnet, daß der Nickel-Aluminium-Zirkondioxid enthaltende Trägerkatalysator 6 bis 80, insbesondere 15 bis 65, bevorzugt 35 bis 50 Gew.-Teile Trägermaterial je 100 Gew.-Teile Ni enthält.

11. Verfahren nach einem oder mehreren der Ansprüche 1 bis 10, dadurch gekennzeichnet, daß die Raumgeschwindigkeit ausgedrückt als Volumen flüssiger Einsatzstoff zu Volumen Katalysator pro Stunde (V/Vh) in der ersten Stufe 0,3 bis 2,0, insbesondere 0,5 bis 1,8, bevorzugt 0,6 bis 1,5 und in der zweiten Stufe 0,5 bis 2,5, insbesondere 0,7 bis 1,8, bevorzugt 0,8 bis 1,5 beträgt.

## Claims

1. A process for the preparation of alcohols by reaction of organic carbonyl compounds at elevated temperature and, if appropriate, elevated pressure with hydrogen in two stages, in which the organic carbonyl compounds are fed in the first stage as a gas to a copper-containing catalyst and in the second stage as a liquid to a nickel-containing catalyst, which comprises reacting 80 to 99.5 % of theory of the organic carbonyl compounds in the first stage and in the second stage by means of a supported catalyst containing nickel/alumina/zirconium dioxide.

2. The process as claimed in claim 1, wherein the carbonyl compounds used are ketones, ketone derivatives, aldehydes and aldehyde derivatives.

3. The process as claimed in one or more of claims 1 to 2, wherein the carbonyl compounds used are aldehydes and aldehyde derivatives, in particular saturated and unsaturated aliphatic aldehydes.

4. The process as claimed in one or more of claims 1 to 3, wherein the carbonyl compounds used are n-butanal and 2-ethylhexenal.

5. The process as claimed in one or more of claims 1 to 4, wherein the copper-containing catalyst contains 15 to 85 % by weight of copper, 0.2 to 20 % by weight of MgO, 0.03 to 12 % by weight of Cr$_2$O$_3$ and 5 to 80 % by weight of SiO$_2$ as support, in each case relative to the composition of the catalyst.

6. The process as claimed in one or more of claims 1 to 5, wherein in the first stage the temperature is 100 to 200°C and the pressure 0.05 to 2.0 MPa and in the second stage the temperature is 60 to 150°C and the pressure 0.1 to 25 MPa.

7. The process as claimed in one or more of claims 1 to 6, wherein in the first stage 90 to 99.0, in particular 93 to 98, % of theory of the carbonyl compounds are converted.

8. The process as claimed in one or more of claims 1 to 7, wherein the hydrogenation catalyst contains 20 to 90 % by weight of nickel, relative to the composition of the catalyst, and 1 to 30 parts by weight of alumina and 0.5 to 20 parts by weight of zirconium dioxide, in each case relative to 100 parts by weight of Ni as coprecipitate on a support material.

9. The process as claimed in one or more of claims 1 to 8, wherein the supported catalyst containing nickel/alumina/zirconium dioxide contains, as support material, activated carbon, clay, pumice, $\gamma$-Al$_2$O$_3$, SiO$_2$, silica gel, kieselguhr and/or siliceous earth, in particular SiO$_2$, silica gel, kieselguhr and/or siliceous earth, preferably kieselguhr and/or SiO$_2$ in the form of precipitated silica.

10. The process as claimed in one or more of claims 1 to 9, wherein the supported catalyst containing nickel/alumina/zirconium dioxide contains 6 to 80, in particular 15 to 65, preferably 35 to 50, parts by weight of support material per 100 parts by weight of Ni.

11. The process as claimed in one or more of claims 1 to 10, wherein the space velocity expressed as the volume of liquid feed material to the volume of catalyst per hour (V/Vh) is 0.3 to 2.0, in particular 0. 5 to 1. 8, preferably 0. 6 to 1. 5, in the first stage and 0.5 to 2.5, in particular 0.7 to 1.8, preferably 0.8 to 1.5, in the second stage.

**Revendications**

1. Procédé pour la fabrication d'alcools par réaction de composés carbonylés organiques à température élevée et éventuellement sous pression élevée avec l'hydrogène en deux étapes, dans lequel les composés carbonylés organiques sont envoyés dans la première étape sous forme gazeuse sur un catalyseur au cuivre et dans la seconde étape sous forme liquide sur un catalyseur au nickel, caractérisé en ce que les composés carbonylés organiques sont mis à réagir dans la première étape à 80-99,5 % de la théorie et dans la seconde étape au moyen d'un catalyseur au nickel-oxyde d'aluminium-dioxyde de zirconium sur support.

2. Procédé selon la revendication 1, caractérisé en ce que l'on utilise comme composés carbonylés des cétones, des dérivés de cétones, des aldéhydes et des dérivés d'aldéhydes.

3. Procédé selon une ou plusieurs des revendications 1 à 2, caractérisé en ce que l'on utilise comme composés carbonylés des aldéhydes et des dérivés d'aldéhydes, en particulier des aldéhydes aliphatiques saturés et insaturés.

4. Procédé selon une ou plusieurs des revendications 1 à 3, caractérisé en ce que l'on utilise comme composés carbonylés le n-butanal et le 2-éthylhexénal.

5. Procédé selon une ou plusieurs des revendications 1 à 4, caractérisé en ce que le catalyseur au cuivre contient 15 à 85 % en poids de cuivre, 0,2 à 20 % en poids de MgO, 0,03 à 12 % en poids de $Cr_2O_3$ et 5 à 80 % en poids de $SiO_2$ comme support, rapportés chaque fois à la composition du catalyseur.

6. Procédé selon une ou plusieurs des revendications 1 à 5, caractérisé en ce que la température est de 100 à 200°C et la pression de 0,05 à 2,0 MPa dans la première étape et la température est de 60 à 150°C et la pression de 0,1 à 25 MPa dans la seconde étape.

7. Procédé selon une ou plusieurs des revendications 1 à 6, caractérisé en ce que l'on fait réagir les composés carbonylés dans la première étape à 90-99,0 % de la théorie, en particulier 93 à 98 %.

8. Procédé selon une ou plusieurs des revendications 1 à 7, caractérisé en ce que le catalyseur d'hydrogénation contient 20 à 90 % en poids de nickel par rapport à la composition du catalyseur ainsi que 1 à 30 parties en poids d'oxyde d'aluminium et 0,5 à 20 parties en poids de dioxyde de zirconium sous forme de coprécipité sur une matière de support, rapportés chaque fois à 100 parties en poids de nickel.

9. Procédé selon une ou plusieurs des revendications 1 à 8, caractérisé en ce que le catalyseur nickel-oxyde d'aluminium-dioxyde de zirconium sur support contient comme matière de support du charbon actif, de l'argile, de la pierre ponce, $Al_2O_3$ γ, $SiO_2$, du gel de silice, de la terre d'infusoires et/ou de la silice, en particulier $SiO_2$, du gel de silice, de la terre d'infusoires et/ou de la silice, de préférence de la terre d'infusoires et/ou $SiO_2$ sous forme d'acide silicique précipité.

10. Procédé selon une ou plusieurs des revendications 1 à 9, caractérisé en ce que le catalyseur au nickel-oxyde d'aluminium-dioxyde de zirconium sur support contient 6 à 80 parties en poids de matière de support, en particulier 15 à 65, de préférence 35 à 50 parties en poids, pour 100 parties en poids de Ni.

11. Procédé selon une ou plusieurs des revendications 1 à 10, caractérisé en ce que la vitesse spatiale horaire, exprimée en volume de charge liquide par volume de catalyseur et par heure (V.S.H.L.en h$^{-1}$) est de 0,3 à 2,0, en particulier 0,5 à 1,8, de préférence 0,6 à 1,5 dans la première étape et de 0,5 à 2,5, en particulier 0,7 à 1,8, de préférence 0,8 à 1,5 dans la seconde étape.